# EUROPÄISCHE PATENTSCHRIFT

(11) **EP 4 245 283 B1**
(45) Veröffentlichungstag und Bekanntmachung des Hinweises auf die Patenterteilung: **10.12.2025**
(21) Anmeldenummer: 22162404.2
(22) Anmeldetag: 16.03.2022
(51) Int. Cl.: A61H 39/08

(54) **MICRONEEDLING-HANDGERÄT ZUM LOKALEN STIMULIEREN EINER HAUT, HAUTSTIMULATIONSEINRICHTUNG UND ARTIKEL**
HANDHELD MICRONEEDLING DEVICE FOR THE LOCAL STIMULATION OF A SKIN, SKIN STIMULATION DEVICE AND ARTICLE
APPAREIL PORTATIF DE MICRO-AIGUILLAGE PERMETTANT DE STIMULER LOCALEMENT LA PEAU, DISPOSITIF DE STIMULATION DE LA PEAU ET ARTICLE

(43) Veröffentlichungstag der Anmeldung: 20.09.2023
(73) Patentinhaber: MT.DERM GmbH, 12307 Berlin (DE)
(72) Erfinder: SCHERKOWSKI, Dirk, 12489 Berlin (DE)
(74) Vertreter: Bittner, Thomas L.

(56) Entgegenhaltungen:
- US-B1- 9 636 491

## Beschreibung

Die Erfindung betrifft ein Microneedling-Handgerät zum lokalen Stimulieren einer Haut, eine Hautstimulationseinrichtung für ein Microneedling-Handgerät und einen Artikel.

### Hintergrund

Microneedling kann vorsehen, die Haut mit mehreren Stechnadeln in einem flächigen Hautabschnitt gleichzeitig lokal aufzustechen. Die mehreren Stechnadeln sind an einem Microneedling-Hautstechwerkzeug eines Handgeräts über eine Anwendungsfläche verteilt angeordnet, zum Beispiel an einer Nadelplatte.

Das Microneedling verursacht aufgrund des lokalen Aufstechens feinste Verletzungen der Haut, die die Haut als Wunden registriert. In der Haut werden dann verschiedene Botenstoffe und Wachstumsfaktoren ausgeschüttet, um die Wundheilung anzuregen. Dieser Vorgang regt auch die Produktion von Kollagen, Elastin und Hyaluronsäure an, die Straffung und Elastizität der Haut fördern. Microneedling kann mit und ohne Substanz- oder Wirkstoffeintrag in die Haut ausgeführt werden. Die Haut wird lokal aufgestochen, um positive Heilungsreaktionen auszulösen, was wahlweise mittels Substanzeintrag unterstützt werden kann.

Zum Anwenden des Microneedling sind beispielsweise manuelle Hautstecheinrichtungen in stempelartiger Ausführung bekannt, bei der das Microneedling-Hautstechwerkzeug mit einem Stempel gebildet ist, bei dem über eine Anwendungsfläche verteilt mehrere Stechnadeln angeordnet sind. Bei anderen manuellen Handgeräten sind die Stechnadeln über die Mantelfläche einer Walze verteilt, die zum lokalen Aufstechen der Haut über diese gerollt wird.

Weiterhin sind Microneedling-Handgeräte in Form eines sogenannten Pens bekannt, bei dem ein Microneedling-Hautstechwerkzeug einer Hautstecheinrichtung mit einer Nadelplatte gebildet ist, an der die mehreren Stechnadeln im Bereich einer Anwendungsfläche angeordnet sind. Das Microneedling-Hautstechwerkzeug wird mit Hilfe eines elektrischen Motors einer Antriebseinrichtung automatisiert vor- und zurückbewegt. Mit Hilfe des elektrischen Motors der Antriebseinrichtung wird eine Antriebskraft bereitgestellt, die auf das Microneedling-Hautstechwerkzeug eingekoppelt wird, um die Nadelplatte mit der Anwendungsfläche und den hierauf flächig verteilten Stechnadeln vor und zurück zu bewegen.

Ein Microneedling-Handgerät ist zum Beispiel aus dem Dokument US 2017 / 0354810 A1 bekannt. US 9,636,491 B1 beschreibt ein dermatologisches Microneedlinggerät.

### Zusammenfassung

Aufgabe der Erfindung ist es, ein Microneedling-Handgerät zum lokalen Stimulieren und / oder Regenerieren einer Haut anzugeben, welches eine erweiterte Anwendung des Microneedlings ermöglicht.

Gelöst wird die Aufgabe durch ein Microneedling-Handgerät zum lokalen Stimulieren und / oder Regenerieren einer Haut nach Anspruch 1. Weiterhin sind eine Hautstimulationseinrichtung für ein Microneedling-Handgerät sowie ein Artikel nach den nebengeordneten Ansprüchen 15 und 16 geschaffen. Ausgestaltungen sind Gegenstand von abhängigen Unteransprüchen.

Nach einem Aspekt ist ein Microneedling-Handgerät zum lokalen Stimulieren einer Haut geschaffen, welches Folgendes aufweist: ein Gehäuse; eine Antriebseinrichtung, die in dem Gehäuse angeordnet und eingerichtet ist, eine Antriebskraft bereitzustellen; eine Hautstimulationseinrichtung, die in dem Gehäuse angeordnet ist; ein Microneedling-Stimulationswerkzeug, das an der Hautstimulationseinrichtung gebildet und mit der Antriebseinrichtung verbunden ist, derart, dass das Microneedling-Stimulationswerkzeug mittels der Antriebskraft im Betrieb mit einer Wiederholfrequenz vor und zurück bewegbar ist; und eine Nadelplatte, die an dem Microneedling-Stimulationswerkzeug gebildet ist und bei der über eine vorderseitige Anwendungsfläche verteilt mehrere nicht-stechende Stimulationsnadeln mit einer stumpfen Nadelspitze angeordnet sind. Das Microneedling-Stimulationswerkzeug ist mittels der Antriebskraft im Betrieb mit der Wiederholfrequenz zwischen einer vorderen Arbeitsstellung und einer im Vergleich hierzu zurückgestellten hinteren Arbeitsstellung vor und zurück bewegbar.

Nach einem weiteren Aspekt ist eine Hautstimulationseinrichtung mit einem Gehäuse geschaffen, die zum Ausbilden eines Microneedling-Handgeräts an eine Antriebseinrichtung koppelbar ist und Folgendes aufweist: ein Microneedling-Stimulationswerkzeug, das an der Hautstimulationseinrichtung gebildet und mit der Antriebseinrichtung verbindbar ist, derart, dass das Microneedling-Stimulationswerkzeug mittels einer von der Antriebseinrichtung bereitgestellten Antriebskraft im Betrieb mit einer Wiederholfrequenz vor und zurück bewegbar ist; und eine Nadelplatte, die an dem Microneedling-Stimulationswerkzeug gebildet ist und bei der über eine vorderseitige Anwendungsfläche verteilt mehrere nicht-stechende Stimulationsnadeln mit einer stumpfen Nadelspitze angeordnet sind. Das Microneedling-Stimulationswerkzeug ist zwischen einer vorderen Arbeitsstellung und einer im Vergleich hierzu zurückgestellten hinteren Arbeitsstellung vor und zurück bewegbar ist, wobei zumindest die mehreren nicht-stechenden Stimulationsnadeln der Nadelplatte hierbei in der vorderen Arbeitsstellung und der hinteren Arbeitsstellung des Microneedling-Stimulationswerkzeugs vollständig außerhalb des Gehäuses und so freiliegend angeordnet sind.

Nach einem weiteren Aspekt ist ein Artikel geschaffen, der Folgendes aufweist: eine Antriebseinrichtung, die in einem Gehäuse angeordnet ist, welches wahlweise ein Handstück ausbildend ausgeführt ist; einen Zahnbürstenaufsatz, welcher zum Ausbilden einer elektrischen Zahnbürste mit dem Gehäuse lösbar und so an die Antriebseinrichtung koppelnd verbunden werden kann; und eine Hautstimulationseinrichtung, welche zum Ausbilden eines Microneedling-Handgeräts mit dem Gehäuse lösbar und so an die Antriebseinrichtung koppelnd verbunden werden kann.

Das Microneedling-Stimulationswerkzeug ist mittels der Antriebskraft im Betrieb mit der Wiederholfrequenz entlang einer Bewegungsrichtung vor und zurück bewegbar. Entlang der Bewegungsrichtung wird ein Bewegungshub zwischen Stellungen des Microneedling-Stimulationswerkzeugs ausgeführt.

Mithilfe der nicht-stechenden Ausführung der Nadeln ist ein Handgerät bereitgestellt, welches eine Microneedling-Behandlung (Microneedling) der Haut ermöglicht, ohne diese lokal aufzustechen, insbesondere für ein Microneedling oder ein Microneedling-ähnliches Behandeln in Hautbereichen, in denen die obere Hautschicht (Epidermis) dünner oder feiner ist als in anderen Hautbereichen, zum Bespiel im Bereich von Hautabschnitte im Gesicht, beispielsweise um die Augen herum.

Die nicht-stechenden Stimulationsnadeln können auf der Nadelplatte aufrechtstehend ausgerichtet sein.

Beim Bewegen zwischen der vorderen Arbeitsstellung und der hinteren Arbeitsstellung können in einer Ausführung zumindest die mehreren nicht-stechenden Stimulationsnadeln der Nadelplatte in der vorderen Arbeitsstellung und der hinteren Arbeitsstellung des Microneedling-Stimulationswerkzeugs vollständig außerhalb des Gehäuses und so freiliegend angeordnet sein. Das Microneedling-Handgerät ist in einer beispielhaften Ausgestaltung so konstruktiv eingerichtet, ein weiter verbessertes Microneedling zu ermöglichen. Da zumindest die mehreren nicht-stechenden Stimulationsnadeln der Nadelplatte an dem Microneedling-Stimulationswerkzeug sowohl in der vorderen Arbeitsstellung wie auch in der hinteren Arbeitsstellung jeweils vollständig außerhalb des wahlweise ein- oder mehrstückigen Gehäuses, insbesondere außerhalb eines Gehäusebauteils der Hautstimulationseinrichtung, und so freiliegend angeordnet sind, kann der lokal zu stimulierende Hautabschnitt aufgrund der repetierenden Bewegung der Nadelplatte mit den mehreren nicht-stechenden Stimulationsnadeln selbst in Schwingungen versetzt werden, wird also nicht vom Gehäuse des Microneedling-Handgeräts hieran gehindert, was ein effizientes und möglichst schmerzfreies Anwenden der mehreren nicht-stechenden Stimulationsnadeln auf der Hautoberfläche ermöglicht, ohne in die Haut einzudringen.

Im Betrieb kann in einer beispielhaften Ausführung weiterhin zumindest die vorderseitige Anwendungsfläche sowohl in der vorderen Arbeitsstellung wie auch in der zurückgestellten hinteren Arbeitsstellung des Microneedling-Stimulationswerkzeugs vollständig außerhalb des Gehäuses und so freiliegend angeordnet sein, insbesondere außerhalb eines von Gehäuseabschnitten des Gehäuses zumindest teilweise umgebenen Gehäuseinnenraum. Auf diese Weise ist das Microneedling-Handgerät ergänzend konstruktiv eingerichtet, im Betrieb beim Microneedling das freie Schwingen des bearbeiteten Hautabschnitts zuzulassen.

Die vorderseitige Anwendungsfläche mit den hierauf verteilt angeordneten mehreren nicht-stechenden Stimulationsnadeln kann, zumindest in der vorderen Arbeitsstellung, zu wenigstens einer Bezugsrichtung aus der folgenden Gruppe schräg gestellt oder geneigt sein: Längsrichtung des Gehäuses, Bewegungsrichtung des Microneedling-Stimulationswerkzeugs beim Verlagern zwischen der vorderen und der zurückgestellten hinteren Arbeitsstellung und Bewegungsrichtung der Nadelplatte beim Verlagern zwischen der vorderen und der zurückgestellten hinteren Arbeitsstellung.

Die vorderseitige Anwendungsfläche mit den hierauf verteilt angeordneten mehreren nicht-stechenden Stimulationsnadeln kann sowohl in der vorderen wie auch in der im Vergleich hierzu zurückgestellten hinteren Arbeitsstellung schräg gestellt sein. Hierbei kann die Schräg- oder Neigungsstellung auch während der Bewegung zwischen der vorderen und der hinteren Arbeitsstellung ausgebildet sein.

Die vorderseitige Anwendungsfläche kann mit der Längsrichtung des Gehäuses zum Beispiel einen Winkel zwischen etwa 30 Grad und < 90 Grad einschließen, bevorzugt zwischen etwa 30 Grad und etwa 85 Grad und weiter bevorzugt zwischen etwa 45 Grad und etwa 80 Grad.

Bei dem Microneedling-Handgerät kann eine Schutz- oder Abdeckkappe vorgesehen sein, die am Gehäuse lösbar und die Nadelplatte abdeckend angeordnet ist, wobei an der Schutzkappe außen ein Funktionswerkzeug angeordnet ist. Zum Beispiel können an der Schutzkappe außen, insbesondere im Bereich einer Fronfläche, mehrere Hautmassagevorsprünge angeordnet sein, die auf der Außenfläche der Schutzkappe verteilt angeordnet sind. Zum Beispiel können die Hautmassagevorsprünge ein abgerundetes Kopfteil aufweisen, beispielsweise mit einem Kugelkopf.

An der Abdeckkappe, welche im aufgesetzten Zustand die mehreren nicht-stechenden Stimulationsnadeln abdeckt, ist so ein Funktionswerkzeug angeordnet, welches mit einer Anordnung von Funktionselementen gebildet sein kann, bei denen es sich beispielsweise um Massageelemente handelt. Die Abdeckkappe ist so als Multifunktionskappe ausführbar.

An der Abdeckkappe können Öffnungen vorgesehen sein. Zum Beispiel sind die Öffnungen im Bereich einer Mantelfläche der Abdeckkappe angeordnet und bilden so seitliche Öffnungen. Alternativ oder ergänzend können solche Öffnungen bei anderen Ausführungsformen im Bereich der Frontfläche angeordnet sein. Zum Beispiel können die Öffnungen dazu dienen, für die Nadelplatte mit den mehreren Stimulationsnadeln bei aufgesetzter Abdeckkappe eine Gassterilisation durchzuführen. Seitliche Öffnungen haben den Vorteil, dass der Eintritt von Staubpartikeln zu der Nadelplatte und hierdurch verursachte Staubablagerungen gemindert werden.

Bei dem Microneedling-Stimulationswerkzeug kann in alternativen Ausgestaltungen die Nadelplatte frei von einem die Nadelplatte zumindest teilweise umgreifenden Gehäuseabschnitt des Gehäuses gebildet sein.

Die Nadelplatte kann an einem Gehäuseabschnitt des Gehäuses angeordnet sein, welcher im Betrieb beim Vor- und Zurückbewegen des Microneedling-Stimulationswerkzeugs mit diesem zusammen mitbewegt wird. Hierbei kann es sich um ein vorderes Gehäuseteil des Gehäuses handeln. Die Nadelplatte kann hierbei auf einer Außenseite des Gehäuseabschnitts gebildet sein, mit der die Anwendungsfläche bereitgestellt ist. Die Nadelplatte kann an einem vorderen Gehäuseabschnitt des Gehäuses angeformt sein, welcher im Betrieb zusammen mit dem Microneedling-Stimulationswerkzeug vor und zurück bewegt wird. Beispielsweise kann die Nadelplatte als eine Frontplatte des Gehäuseabschnitts angeformt sein. Die Nadelplatte kann lösbar oder nicht-lösbar an dem Gehäuseabschnitt angeordnet sein.

Die Nadelplatte kann an dem Gehäuseabschnitt des Gehäuses einstückig angeformt sein. Hierbei kann ein seitlicher Wandabschnitt des Gehäuseabschnitts einstückig mit einer die Anwendungsfläche bereitstellenden Frontplatte einstückig ausgeführt sein. Der Gehäuseabschnitt kann so das Microneedling-Stimulationswerkzeug bildend ausgeführt sein, insbesondere die Nadelplatte.

Das Microneedling-Stimulationswerkzeug kann zusammen mit dem Gehäuseabschnitt von dem Gehäuse lösbar oder abnehmbar sein. Das Microneedling-Stimulationswerkzeug und / oder die Hautstimulationseinrichtung mit dem Microneedling-Stimulationswerkzeug, sei es separat oder gemeinsam mit dem Gehäuseabschnitt, kann mittels einer Steckverbindung oder einer Schraubverbindung lösbar montiert sein. So kann das Microneedling-Stimulationswerkzeug gemeinsam mit dem Gehäuseabschnitt auf einen zugeordneten Gehäuseabschnitt aufsteckbar sein, insbesondere einen Gehäuseabschnitt, der die Antriebseinrichtung aufnimmt.

Bei verschiedenen Ausführungsformen können die mehreren Stimulationsnadeln im Bereich der Anwendungsfläche auf die Nadelplatte aufgebracht sein. Alternativ kann vorgesehen sein, dass die Stimulationsnadeln in Öffnungen der Nadelplatte angeordnet sind, beispielsweise eingelötet oder eingeklebt sind, wobei sich die Stimulationsnadeln zumindest abschnittsweise durch die Öffnungen hindurch erstrecken können. Es kann vorgesehen sein, dass die Stimulationsnadeln nach dem Durchgang durch die Öffnungen an einem in Bezug auf die Anwendungsfläche der Nadelplatte rückseitigen Nadelhalterelement aufgenommen sind, welches als Teil des Microneedling-Stimulationswerkzeugs oder der Nadelplatte ausbildbar ist.

Alternativ können die Stimulationsnadeln einstückig an der Nadelplatte angeformt sein. So können die Stimulationsnadeln zum Beispiel mittels gestanzter und gebogener Blechelemente gebildet sein.

Stimulationsnadeln im Sinne der vorliegenden Offenbarung sind allgemein nicht-scharfe bzw. nicht-stechende, von der Nadelplatte hervorstehende Volumenelemente, die hinsichtlich Form und Festigkeit für eine wiederholte (nicht-stechende) Anregung der Haut(schichten) eingerichtet sind. Die Stimulationsnadeln sind bevorzugt aus einem Vollmaterial, im Unterschied zur Kanüle (Hohlnadel).

Ein vorderes Gehäuseteil, welches der Hautstimulationseinrichtung zugeordnet ist, kann rückwärtig hülsenförmig gestaltet sein und einen hinteren Gehäuseteil zumindest teilweise umschließen oder umgreifen, welcher der Antriebseinrichtung zugeordnet ist. Bei dieser oder anderen Ausführungsformen kann mit dem vorderen Gehäuseteil, welches vom hinteren Gehäuseteil abnehmbar sein kann, ein Gehäusebauteil der Hautstimulationseinrichtung gebildet sein, zum Beispiel ein Modulgehäuse eines Stimulationsmoduls.

Die Schutzkappe kann auf das vorderseitige Gehäuseende lösbar aufgesetzt sein.

Die Hautstimulationseinrichtung kann an dem Gehäuse lösbar angeordnet sein. Auf diese Weise kann das Microneedling-Stimulationswerkzeug mit einem zugeordneten Gehäuseteil wechselbar an dem Gehäuse angeordnet werden.

Die Antriebseinrichtung kann mit einer Antriebseinrichtung für eine elektrische Zahnbürste gebildet sein, welche eingerichtet ist, hieran wahlweise die Hautstimulationseinrichtung und einen mittels der Antriebseinrichtung betreibbaren Zahnbürstenaufsatz lösbar zu koppeln. Antriebseinrichtungen für elektrische Zahnbürsten sind als solche in verschiedenen Ausführungen bekannt. Das Gehäuse der Antriebseinrichtung bildet häufig ein Handstück der elektrischen Zahnbürste, welches der Nutzer beim Benutzen umgreift. Die Antriebseinrichtung stellt eine Antriebskraft / -bewegung für den elektrischen Betrieb des Zahnbürstenaufsatzes bereit. Der Zahnbürstenaufsatz ist abnehmbar. Die Antriebseinrichtung der elektrischen Zahnbürste kann dann zum Ausbilden des Microneedling-Handgeräts verwendet werden, indem die Hautstimulationseinrichtung auf die Antriebseinrichtung der elektrischen Zahnbürste lösbar aufgesetzt wird, so dass die bereitgestellte Antriebskraft auf das Microneedling-Stimulationswerkzeug eingeleitet werden kann, derart, dass das Microneedling-Stimulationswerkzeug mittels der Antriebskraft im Betrieb mit einer Wiederholfrequenz vor und zurück bewegt wird.

Das Microneedling-Stimulationswerkzeug kann durch eine Öffnung an einem vorderseitigen Gehäuseende des Gehäuses hindurch in eine im Vergleich zur zurückgestellten hinteren Arbeitsstellung weiter zurückgestellte Nichtarbeitsstellung verlagerbar sein, in welcher zumindest die vorderseitige Anwendungsfläche der Nadelplatte gegenüber der Öffnung zurücksteht. Bei dieser Ausführungsform kann das Microneedling-Stimulationswerkzeug im Nicht-Betrieb in die zurückgestellte Nichtarbeitsstellung verlagert werden, beispielsweise derart, dass die mehreren Stimulationsnadeln hinter einer Öffnungsfläche der (Gehäuse-) Öffnung am vorderseitigen Gehäuseende angeordnet und somit mittels des Gehäuses geschützt sind.

Das Gehäuse kann die Öffnung teilweise oder vollständig umgreifen. In der zurückgestellten Nichtarbeitsstellung kann die Nadelplatte umlaufend teilweise oder vollständig von dem Gehäuse umgeben sein, so dass die Nadelplatte dann im Gehäuseinnenraum angeordnet ist.

Die Nadelplatte kann beim Verlagern durch die (Gehäuse-)Öffnung hindurch ein- oder mehrseitig von einem die Öffnung umgebenden Rand beabstandet sein. Bei dieser Ausführungsform wird die Nadelplatte in dem ein- oder mehrseitig ausgebildeten Abstandsbereichen berührungskontaktfrei durch die Öffnung hindurch verlagert. Insbesondere findet so in den Abstandsbereichen keine Führung der Nadelplatte am Gehäuse statt. Die Beabstandung zwischen der Nadelplatte und dem umgebenden Gehäuse kann um die Nadelplatte herum umlaufend durchgehend ausgebildet sein.

Die Nadelplatte kann in der weiter zurückgestellten Nichtarbeitsstellung beabstandet von die Nadelplatte umgebenden Gehäuseabschnitten des Gehäuses freiliegend angeordnet sein. Die Nadelplatte ist hier berührungskontaktfrei in der weiter zurückgestellten Nichtarbeitsstellung angeordnet.

Das Microneedling-Handgerät kann mit einem wiederverwendbaren Antriebsmodul, in welchem die Antriebseinrichtung angeordnet ist, und einem Einwegmodul gebildet sein, welches mit dem Antriebsmodul lösbar verbunden ist und in dem die Hautstimulationseinrichtung angeordnet ist. Das Antriebsmodul und das Einwegmodul können mit einem jeweils zugeordneten Modulgehäuse gebildet sein, wobei die Modulgehäuse lösbar miteinander gekoppelt oder verbunden werden können.

Die (insbesondere motorische) Antriebseinrichtung kann eingerichtet sein, das Microneedling-Stimulationswerkzeug mittels der hierauf eingeleiteten Antriebskraft im Betrieb mit einer Wiederholfrequenz zwischen etwa 10 Hz und etwa 200 Hz vor und zurück zu bewegen, alternativ mit einer Wiederholfrequenz zwischen etwa 25 Hz und etwa 160 Hz.

Es kann vorgesehen sein, dass zwischen der vorderen Arbeitsstellung und der im Vergleich hierzu zurückgestellten hinteren Arbeitsstellung in einer Bewegungsrichtung des Microneedling-Stimulationswerkzeugs und / oder Längsrichtung des Microneedling-Handgeräts (insbesondere dessen Gehäuse) ein Abstand (Arbeitshublänge) von etwa 0,5 mm bis etwa 5 mm gebildet ist. Alternativ kann vorgesehen sein, dass zwischen der vorderen Arbeitsstellung und der im Vergleich hierzu zurückgestellten hinteren Arbeitsstellung in der Bewegungsrichtung des Microneedling-Stimulationswerkzeugs und / oder Längsrichtung des Microneedling-Handgeräts ein Abstand (Arbeitshublänge) von lediglich etwa 1 mm bis etwa 4 mm gebildet ist. Mittels unterschiedlicher Hublängen ist das Microneedling-Handgerät für unterschiedliche Anwendungen konfigurierbar, zum Beispiel für medizinisches oder kosmetisches Microneedling.

Die Nadelplatte kann in einer Ausgestaltung mit einer Wendeplatte oder einem Wendeeinsatz ausgeführt sein, derart, dass unterschiedliche Microneedling-Stimulationswerkzeuge hieran jeweils wechsel- oder lösbar angeordnet werden können, um unterschiedliche (Microneedling-)Funktionen bereitzustellen und / oder einen Verschleiß des Stimulationswerkzeugs zu kompensieren. Es kann sich auch um Wechseleinsätze handeln, die je nach gewünschter Anwendung an der Nadelplatte lösbar angeordnet werden. Wendeeinsätze können auf beiden Seiten Stimulationswerkzeuge tragen. Die Wechseleinsätze können aus einem Sortiment oder einem abgestuften Satz von Werkzeugeigenschaften auswählbar sein.

Die Anwendungsfläche, auf welcher die mehreren nicht-stechenden Stimulationsnadeln angeordnet sind, kann eine konvex gekrümmte Oberfläche aufweisen, welche die Anwendungsfläche ganz oder nur teilweise erfasst, insbesondere nur in einem mittleren Flächenbereich.

Die vorangehend in Verbindung mit dem Microneedling-Handgerät erläuterten Ausgestaltungen können im Zusammenhang mit der Hautstimulationseinrichtung und / oder dem Artikel entsprechend vorgesehen sein.

### Beschreibung von Ausführungsbeispielen

Im Folgenden werden weitere Ausführungsbeispiele unter Bezugnahme auf Figuren einer Zeichnung erläutert. Hierbei zeigen:
- Fig. 1: eine schematische perspektivische Darstellung eines Microneedling-Handgeräts;
- Fig. 2: eine schematische perspektivische Darstellung des Microneedling-Handgeräts aus Fig. 1., wobei ein Stimulationsmodul mit einem Microneedling-Stimulationswerkzeug gelöst ist;
- Fig. 3: eine schematische perspektivische Darstellung eines vorderen Abschnitts des Microneedling-Handgeräts aus Fig. 1, wobei das Microneedling-Stimulationswerkzeug mit Nadelplatte in einer vorderen Arbeitsstellung angeordnet ist;
- Fig. 4: eine schematische perspektivische Darstellung des vorderen Abschnitts des Microneedling-Handgeräts, wobei das Microneedling-Stimulationswerkzeug mit Nadelplatte in einer zurückgestellten hinteren Arbeitsstellung angeordnet ist;
- Fig. 5: eine schematische perspektivische Darstellung des vorderen Abschnitts des Microneedling-Handgeräts, wobei vorderseitig auf das Microneedling-Stimulationswerkzeug die Nadelplatte abdeckend eine Abdeck- oder Schutzkappe aufgesetzt ist;
- Fig. 6: eine schematische perspektivische Darstellung des vorderen Abschnitts des Microneedling-Handgeräts teilweise im Schnitt mit aufgesetzter Abdeck- oder Schutzkappe;
- Fig. 7: eine schematische perspektivische Darstellung des vorderen Abschnitts des Microneedling-Handgeräts teilweise im Schnitt mit abgenommener Abdeck- oder Schutzkappe;
- Fig. 8: eine schematische perspektivische Darstellung einer elektrischen Zahnbürste;
- Fig. 9: eine schematische perspektivische Darstellung eines weiteren Microneedling-Handgeräts;
- Fig. 10: eine schematische perspektivische Darstellung eines vorderen Gehäuseteils des Microneedling-Handgeräts aus Fig. 9 im Schnitt;
- Fig. 11: eine schematische perspektivische Darstellung des vorderen Gehäuseteils des Microneedling-Handgeräts aus Fig. 9, wobei die Nadelplatte des Microneedling-Stimulationswerkzeugs in einer eingefahrenen Nichtarbeitsstellung angeordnet ist;
- Fig. 12: eine schematische perspektivische Darstellung des vorderen Gehäuseteils des Microneedling-Handgeräts aus Fig. 9, wobei die Nadelplatte des Microneedling-Stimulationswerkzeugs in einer vorderen Arbeitsstellung freiliegend angeordnet ist;
- Fig. 13: eine schematische perspektivische Darstellung des vorderen Gehäuseteils des Microneedling-Handgeräts aus Fig. 9, wobei die Nadelplatte in einer zurückgestellten hinteren Arbeitsstellung freiliegend angeordnet ist;
- Fig. 14: eine schematische perspektivische Darstellung einer Nadelplatte mit einer Anordnung von nicht-stechenden Stimulationsnadeln mit stumpfer Nadelspitze im Bereich einer vorderseitigen Anwendungsfläche von schräg oben;
- Fig. 15: eine schematische perspektivische Darstellung von Nadelplatten unterschiedlicher Größe mit einer Anordnung von nicht-stechenden Stimulationsnadeln mit stumpfer Nadelspitze im Bereich einer vorderseitigen Anwendungsfläche im Schnitt;
- Fig. 16: eine schematische perspektivische Darstellung eines vorderen Abschnitts eines weiteren Microneedling-Handgeräts teilweise im Schnitt;
- Fig. 17: eine schematische perspektivische Darstellung des vorderen Abschnitts des weiteren Microneedling-Handgeräts aus Fig. 15 mit lösbar aufgesetzter Abdeck- oder Schutzkappe;
- Fig. 18: eine schematische perspektivische Darstellung des vorderen Abschnitts des weiteren Microneedling-Handgeräts aus Fig. 16 mit abgenommener Abdeck- oder Schutzkappe und
- Fig. 19: eine schematische perspektivische Darstellung der Abdeck- oder Schutzkappe.

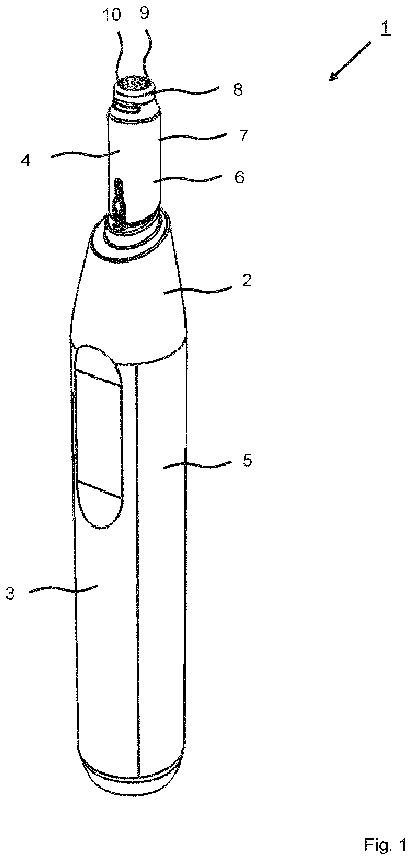

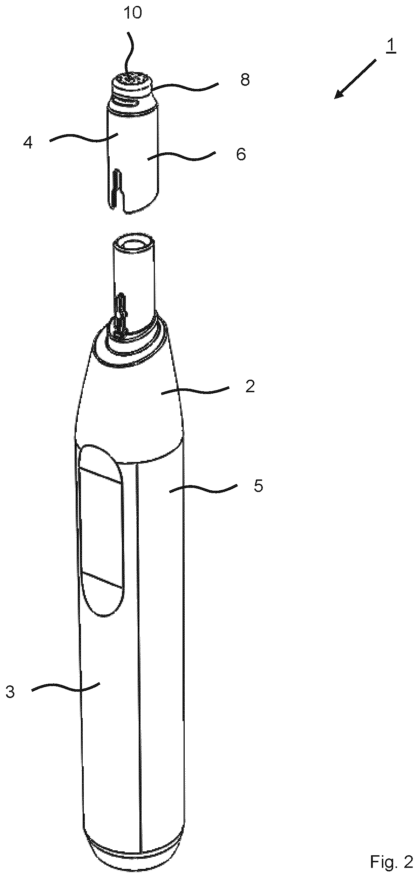

Fig. 1 bis 7 zeigen perspektivische Darstellungen für ein Microneedling-Handgerät 1, welches ein Gehäuse 2 aufweist, welches im gezeigten Beispiel mehrstückig ausgeführt ist. Das Gehäuse 2 ist mit einem hinteren Gehäuseteil 3 und einem vorderen Gehäuseteil 4 gebildet, die einem Antriebsmodul 5 und einer hieran lösbar angeordneten Hautstimulationseinrichtung 6 zugeordnet sind, mit dem wahlweise ein Stimulationsmodul 7 gebildet ist. Gemäß Fig. 2 ist die Hautstimulationseinrichtung 6 und somit wahlweise das Stimulationsmodul 7 von dem Antriebsmodul 5 abnehmbar und auf diese Weise wechselbar. Bei dem gezeigten Beispiel sind Antriebsmodul 5 und Hautstimulationseinrichtung 6 mit einer Steckverbindung lösbar verbunden.

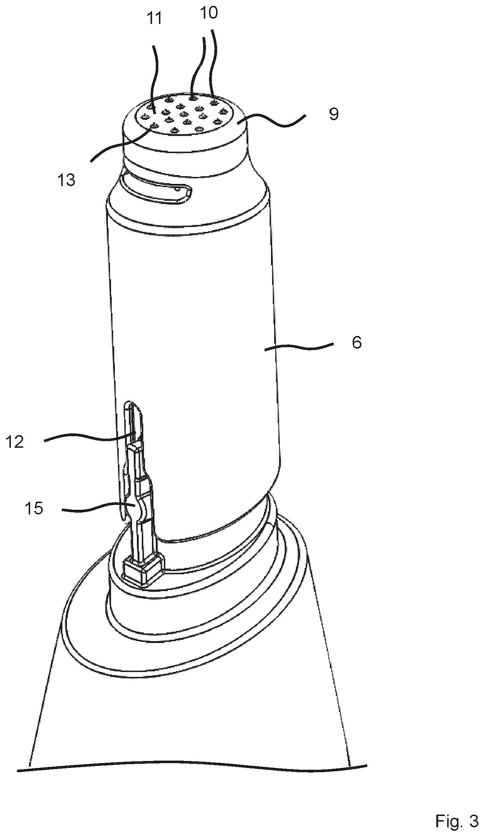

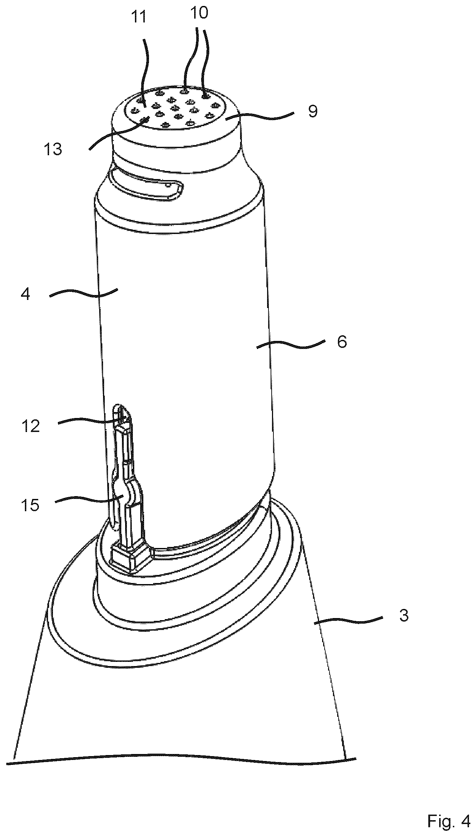

In dem hinteren Gehäuseteil 3, welches dem Antriebsmodul 5 zugeordnet ist, ist eine Antriebseinrichtung angeordnet, beispielsweise ein Elektromotor, mit der eine Antriebsbewegung oder -kraft bereitgestellt wird, die auf ein Microneedling-Stimulationswerkzeug 8 der Hautstimulationseinrichtung 6 gekoppelt wird, um im Betrieb das Microneedling-Stimulationswerkzeug 8 mittels der bereitgestellten Antriebskraft mit einer Wiederholfrequenz in Längsrichtung des Gehäuses 2 vor- und zurück zu bewegen. Auf diese Weise wird das Microneedling-Stimulationswerkzeug 8, welches mit einer Nadelplatte 9 gebildet ist, gemäß den Fig. 3 und 4 zwischen einer vorderen Arbeitsstellung (Fig. 3) und einer hinteren Arbeitsstellung (Fig. 4) repetierend verlagert. Auf diese Weise werden im Betrieb mehrere Stimulationsnadeln 10, die an der Nadelplatte 9 im Bereich einer Anwendungsfläche 11 vorstehen, lokal auf einen zugeordneten Hautabschnitt nicht-stechend angewendet (aufgedrückt) und wieder zurückgezogen. Hierzu sind die mehreren Stimulationsnadeln 10 jeweils mit einer die Haut nicht aufstechenden bzw. stumpfen (nicht-stechenden) Nadelspitze ausgeführt.

Die Nadelplatte 9 mit den mehreren Stimulationsnadeln 10 ist hierbei sowohl in der vorderen als auch in der hinteren Arbeitsstellung gemäß den Fig. 3 und 4 freiliegend, also insbesondere nicht von einem Abschnitt des Gehäuses 2 ganz oder teilweise umgeben und somit außerhalb eines Innenraums 12 des Gehäuses 2 angeordnet. Auf diese Weise kann der lokale Hautabschnitt, welcher mit den mehreren Stimulationsnadeln 10 in Kontakt tritt, aufgrund des Vor- und Zurückbewegens des Microneedling-Stimulationswerkzeugs 8 mit der Nadelplatte 9 frei schwingen, was ein effizientes und möglichst schmerzfreies Anwenden auf der Haut unterstützt.

Im gezeigten Beispiel sind die mehreren Stimulationsnadeln 10 in zugeordneten Öffnungen 13 im Bereich der Anwendungsfläche 11 an der Nadelplatte 9 angeordnet und hierin fixiert, beispielsweise mittels Klebens oder Lötens. In einer anderen Ausführungsform können die mehreren Stimulationsnadeln 10 auf die Anwendungsfläche 11 aufgesetzt werden, beispielsweise mittels Aufschweißens. Alternativ können die Stimulationsnadeln 10 mittels Spritzgussverfahren angeformt sein oder als Einlegebauteile integriert werden. Die mehreren Stimulationsnadeln können oberhalb der Anwendungsfläche 11 alle eine im Wesentlichen gleiche Nadellänge oder unterschiedliche Nadellängen aufweisen, wobei hierbei Gruppen der mehreren Stimulationsnadeln 10 von gleicher Nadellänge sein können.

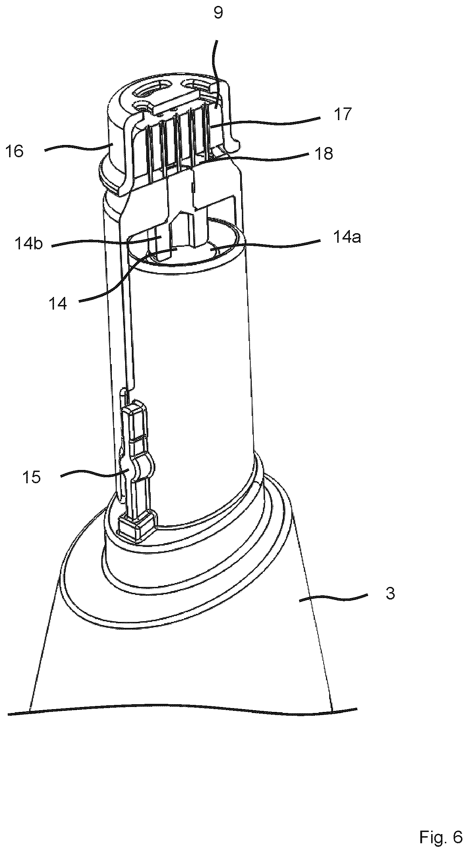

Bei dem gezeigten Beispiel in den Fig. 1 bis 7 ist die Hautstimulationseinrichtung 6, somit wahlweise das Stimulationsmodul 7, mittels Kopplung 14 aufgesteckt, die mit einem antriebsseitigen Kopplungsteil 14a und einem stimulationswerkzeugseitigen Kopplungsteil 14b gebildet ist. Die Kopplung 14 ist beispielweise als eine Magnetkopplung zwischen dem antriebsseitigen Kopplungsteil 14a und dem stimulationswerkzeugseitigen Kopplungsteil 14b ausgebildet (vgl. Fig. 6). Über die Kopplung 14 erfolgt die Einleitung der Antriebskraft / - bewegung zum Vor- und Zurückbewegen der Nadelplatte 9 mit den Stimulationsnadeln 10.

Bei dem gezeigten Beispiel ist eine Verdrehsicherung 15 vorgesehen, welche das Stimulationswerkzeugmodul 6 gegen Verdrehen sichert.

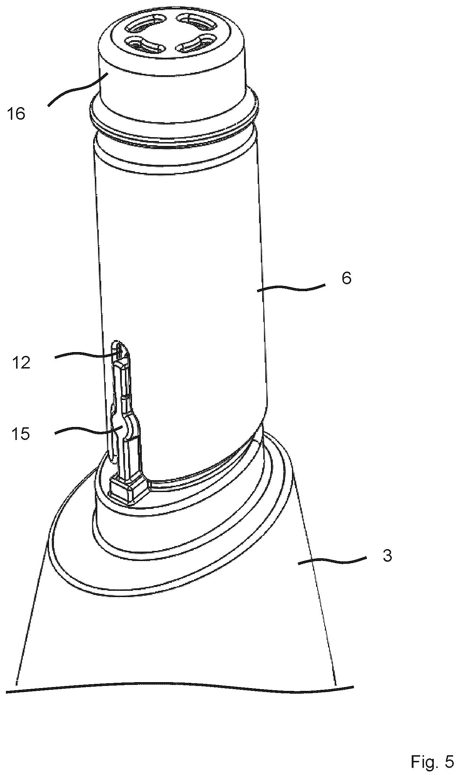

Gemäß Fig. 5 kann die Nadelplatte 9 mit den mehreren Stimulationsnadeln 10 im Nichtbetrieb mittels einer Abdeck- oder Schutzkappe 16 abgedeckt werden, die lösbar aufgesetzt und so zum Betrieb abnehmbar ist. Beim gezeigten Beispiel ist die Abdeck- oder Schutzkappe 16 aufgesteckt.

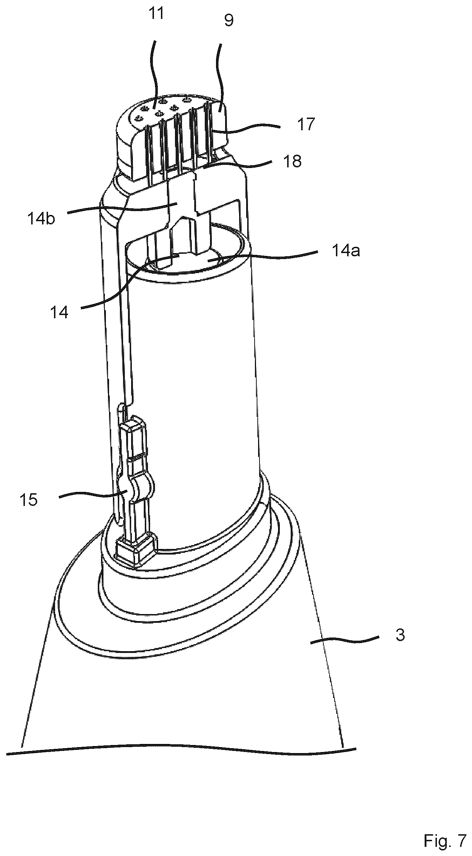

Fig. 6 und 7 zeigen den vorderen Abschnitt des Microneedling-Handgeräts 1 teilweise im Schnitt mit der und ohne die Abdeckkappe 16. Es ergibt sich, dass die mehreren Stimulationsnadeln 10 in zugeordneten Bohrungen 17 der Nadelplatte 9 verlaufen und rückseitig zur Nadelplatte 9 an einem Nadelhalter 18 aufgenommen sind.

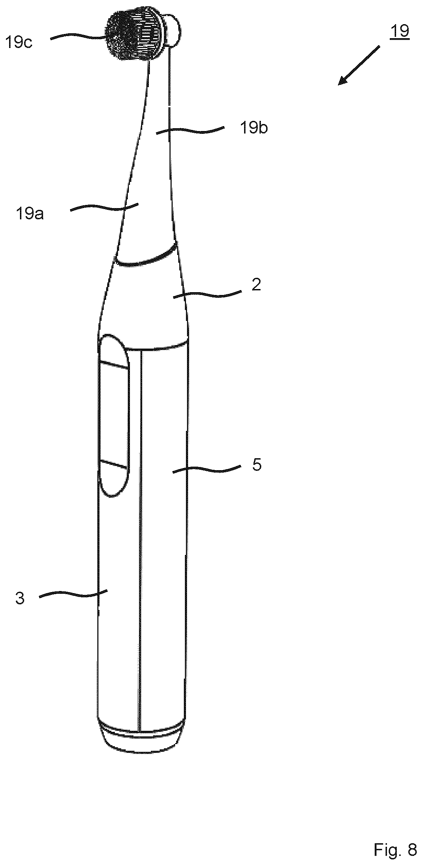

Fig. 8 zeigt eine schematische perspektivische Darstellung einer elektrischen Zahnbürste 19, bei der ein vorderer Gehäuseteil 19a mit einem Zahnbürstenaufsatz 19b gebildet ist, welcher anstelle der Hautstimulationseinrichtung 6 aufgesetzt ist und an die Antriebseinrichtung im hinteren Gehäuseteil 3 des Gehäuses 2 koppelt, so dass die Antriebskraft (Vor- und Zurückbewegung) genutzt wird, um eine Bürstenanordnung 19c im Betrieb zu bewegen. Hautstimulationseinrichtung 6 und Zahnbürstenaufsatz 19b können so wahlweise lösbar aufgesetzt und mit ein und demselben Antriebsmodul 5 betrieben werden, so dass entweder das Microneedling-Handgerät 1 oder die elektrische Zahnbürste 19 bereitgestellt sind.

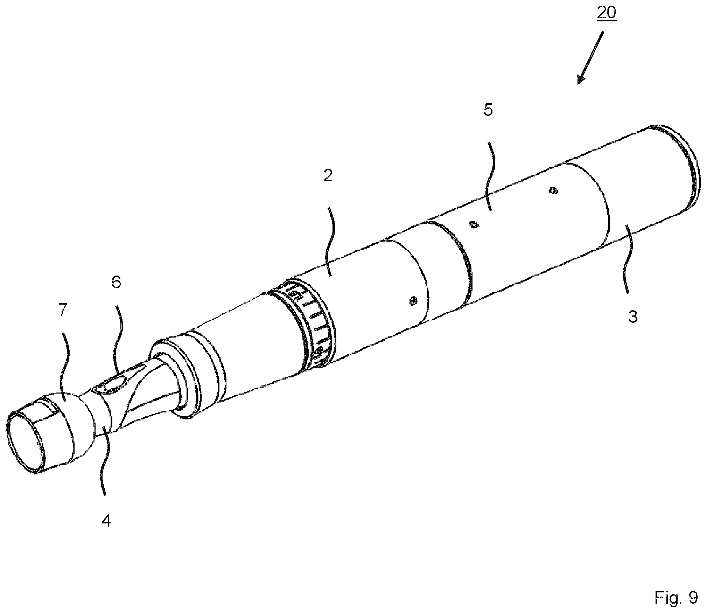

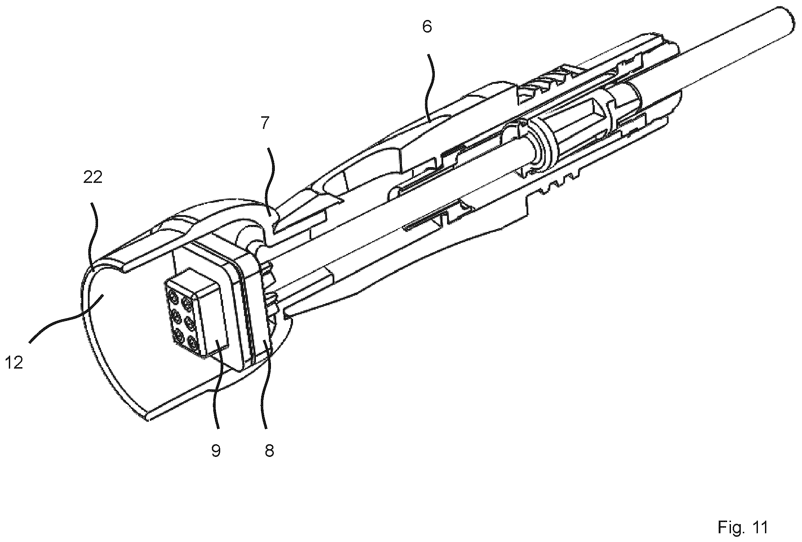

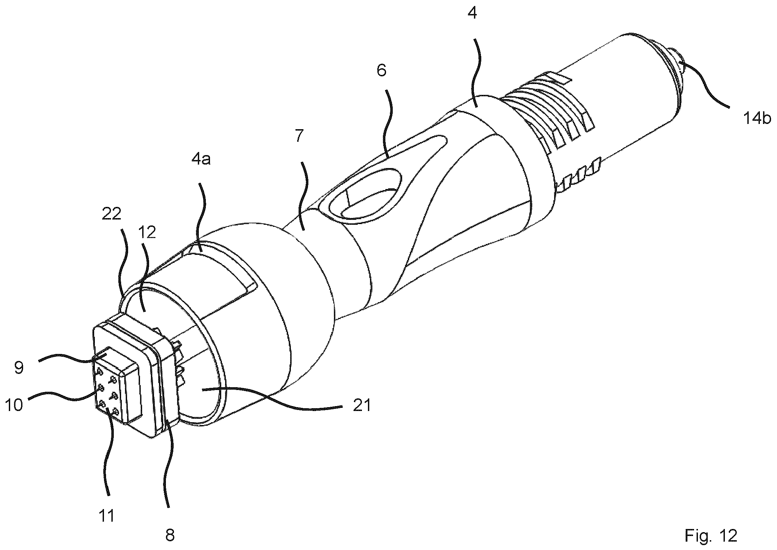

Fig. 9 bis 13 zeigen ein weiteres Microneedling-Handgerät 20, bei dem das Microneedling-Stimulationswerkzeug 8 mit der Nadelplatte 9 durch eine vorderseitige Gehäuseöffnung 21 an der Hautstimulationseinrichtung 6 (Stimulationsmodul 7) zwischen einer eingefahrenen Nichtarbeitsstellung gemäß Fig. 9 bis 11 sowie ausgefahrenen Arbeitsstellungen gemäß den Fig. 11 und 12 verlagerbar ist.

Im Nichtbetrieb kann die Nadelplatte 9 der Hautstimulationseinrichtung 6 mit den hierauf angeordneten mehreren Stimulationsnadeln 10 so hinter die Fläche der Gehäuseöffnung 21 zurückgefahren werden, derart, dass gemäß Fig. 9 sowohl Nadelplatte 9 wie auch Stimulationsnadeln 10 vollständig hinter der Gehäuseöffnung 21 im Innenraum 12 des vorderen Gehäuseteils 4 angeordnet sind, welches bei der gezeigten Ausführungsform der Hautstimulationseinrichtung 6 zugeordnet ist. Am vorderen Gehäuseteils 4 ist die Gehäuseöffnung 21 an einem vorderen Abschnitt 4a gebildet.

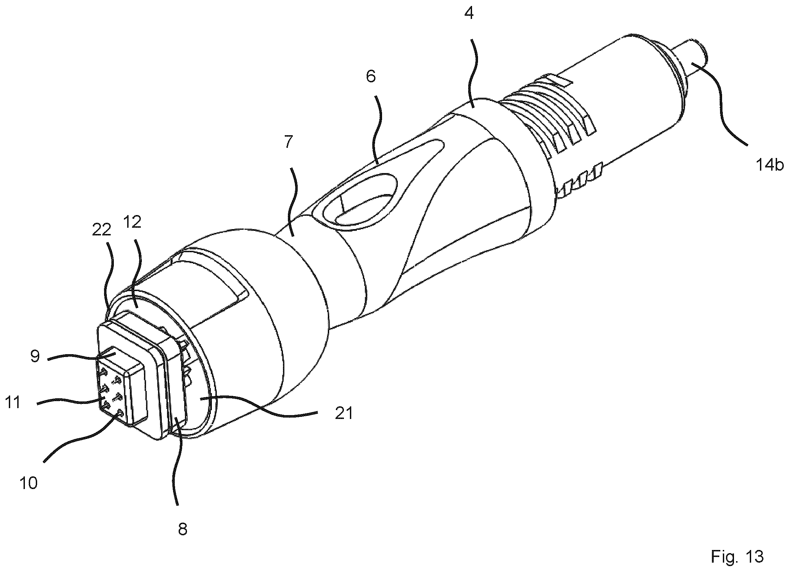

Im Betrieb fürs Microneedling wird die Nadelplatte 9 mit den mehreren Stimulationsnadeln 10 gemäß den Fig. 12 und 13 zwischen der vorderen Arbeitsstellung (vgl. Fig. 12) und der hiergegenüber zurückgestellten hinteren Arbeitsstellung (vgl. Fig. 13) hin und her bewegt, also in Längsrichtung des Gehäuses 2 einem Arbeitshub entsprechend vor und zurück, so dass die Nadelplatte 9 mit den mehreren Stimulationsnadeln 10 sowohl in der vorderen wie auch in der hinteren Arbeitsstellung in Bezug auf die Gehäuseöffnung 21 vor deren Fläche und somit außerhalb des Gehäuses 2 angeordnet ist, insbesondere außerhalb des vorderen Abschnitts 4a des vorderen Gehäuseteils 4, was wiederum ein Anregen des zu bearbeitenden Hautabschnitts zum freien Schwingen ermöglicht, also frei von einer Behinderung dieser Schwingung durch das Gehäuse 2, insbesondere einen die Gehäuseöffnung 21 umgebenden vorderen Gehäuserand 22. Bei dem gezeigten Beispiel ist im Betrieb im Wesentlichen das gesamte Microneedling-Stimulationswerkzeug 8 vor der Gehäuseöffnung 21 angeordnet. Im Nichtbetrieb kann die Nadelplatte 9 dann in die eingefahrene Nichtarbeitsstellung gemäß Fig. 9 bis 10 verlagert werden.

Gemäß dem Beispiel in den Fig. 9 bis 13 ist die Nadelplatte 9 beim Hindurchführen durch die Gehäuseöffnung 21 umlaufend und durchgehend beabstandet vom Gehäuserand 22, also kontakt- und berührungsfrei.

Es kann vorgesehen sein, dass die Nadelplatte 9 innerhalb des vorderen Gehausteils 4 hinter der Gehäuseöffnung 21 mehrere unterschiedliche eingefahrene Nichtarbeitsstellungen einnehmen kann, die sich hinsichtlich ihres jeweiligen Abstands zur Gehäuseöffnung unterscheiden.

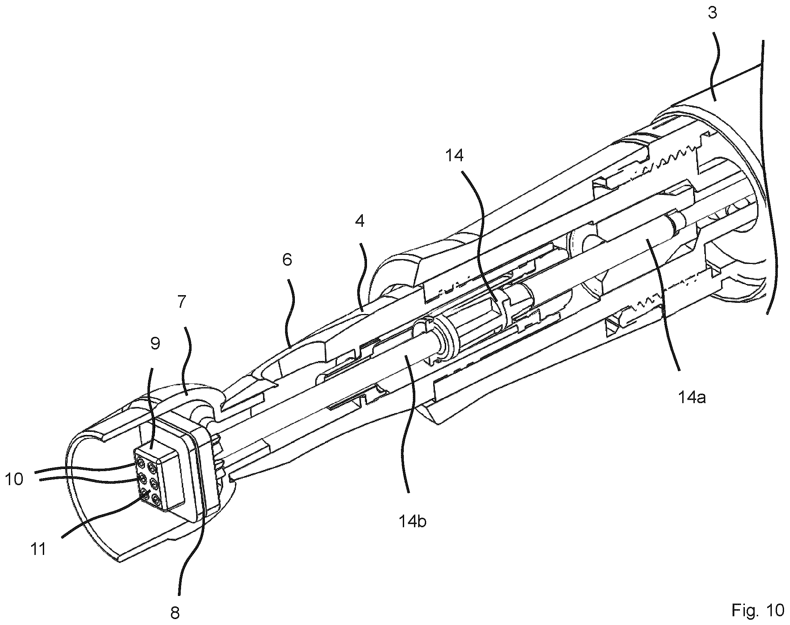

Im Bereich der Kopplung 14 ist das rückseitige, stimulationswerkzeugseitige Kopplungsteil 14b der Hautstimulationseinrichtung 6 gemäß Fig. 10 mit dem hier als Schaftbauteil ausgebildeten antriebsseitigen Kopplungsteil 14a (lösbar) verbunden, um die bereitgestellte Antriebskraft / -bewegung auf das Microneedling-Stimulationswerkzeug 8 mit der Nadelplatte 9 einzukoppeln.

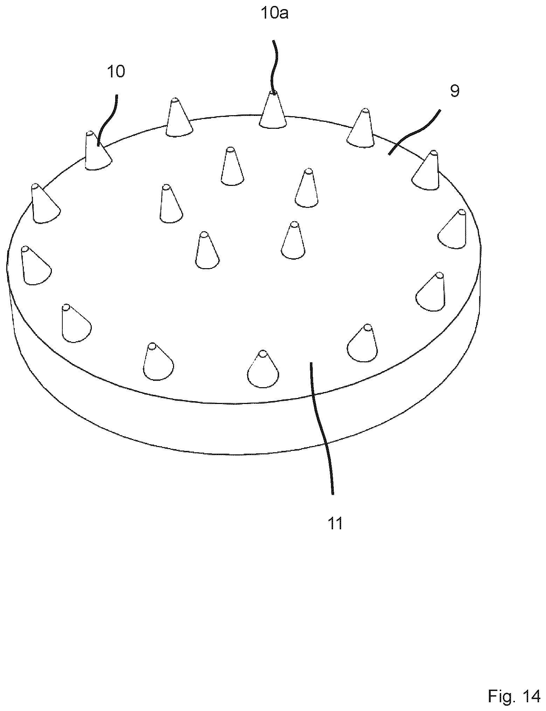

Fig. 14 zeigt eine schematische perspektivische Darstellung einer Ausführung der Nadelplatte 9 mit einer Anordnung der mehreren nicht-stechenden Stimulationsnadeln 10 auf der Anwendungsfläche 11, die jeweils eine stumpfe Nadelspitze 10a aufweisen. Die mehreren Stimulationsnadeln 10 sind bei dem gezeigten Ausführungsbeispiel mit einer Kegelform und einer abgeflachten Nadelspitze ausgebildet. Für gleiche Merkmale werden in den Fig. 14 bis 18 dieselben Bezugszeichen wie in den vorangehenden Figuren verwendet.

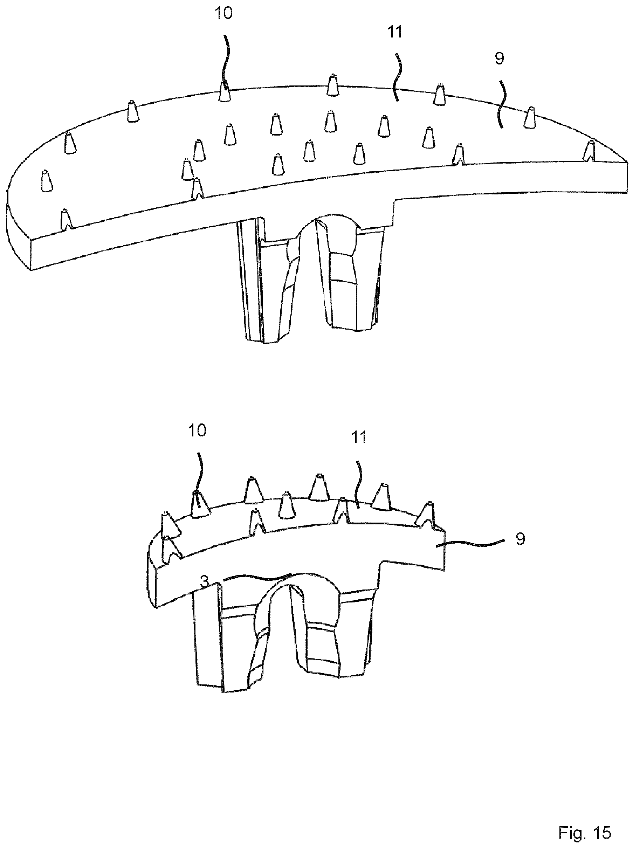

Fig. 15 zeigt eine schematische perspektivische Darstellung von Nadelplatten 9 unterschiedlicher Größe mit einer Anordnung von nicht-stechenden Stimulationsnadeln 10 mit stumpfer Nadelspitze im Bereich der Anwendungsfläche 11 im Schnitt, wobei die Anwendungsfläche 11 konvex gekrümmt ist.

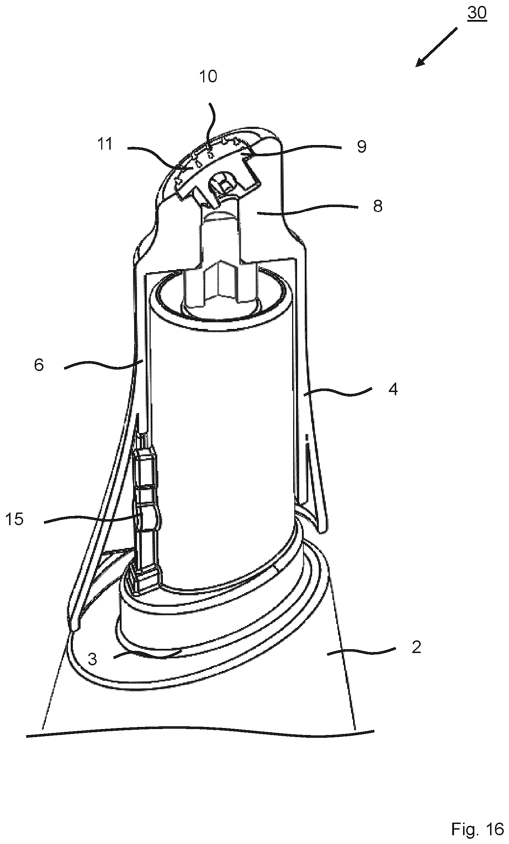

Die Fig. 16 bis 19 zeigen schematische perspektivische Darstellungen eines vorderen Abschnitts eines weiteren Microneedling-Handgerätes 30, bei dem die Anwendungsfläche 11 an der Nadelplatte 9 des Microneedling-Stimulationswerkzeugs 8 schräg gestellt oder geneigt ist in Bezug auf die Längsrichtung des Gehäuses 2, somit im gezeigten Beispiel auch schräg oder geneigt zur Bewegungsrichtung bei der Bewegung des Microneedling-Stimulationswerkzeugs 8 (mit der Nadelplatte 9) zwischen der vorderen und der hinteren Arbeitsstellung.

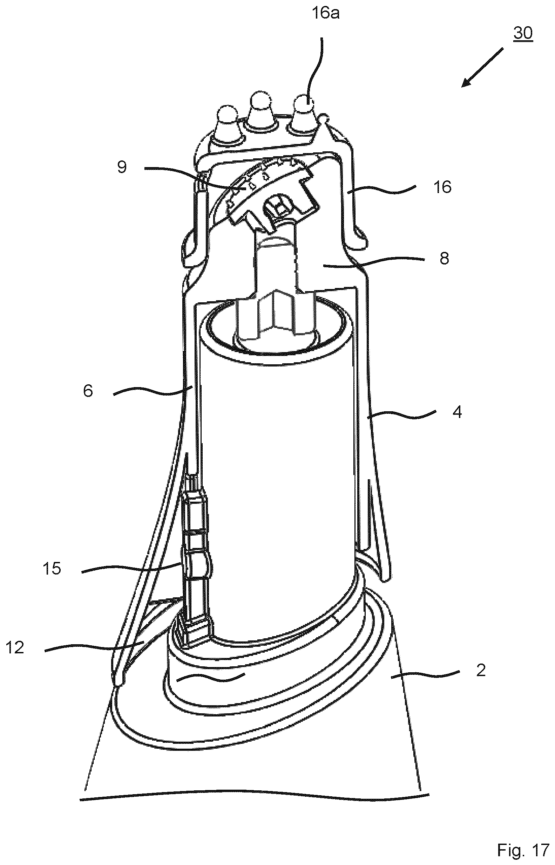

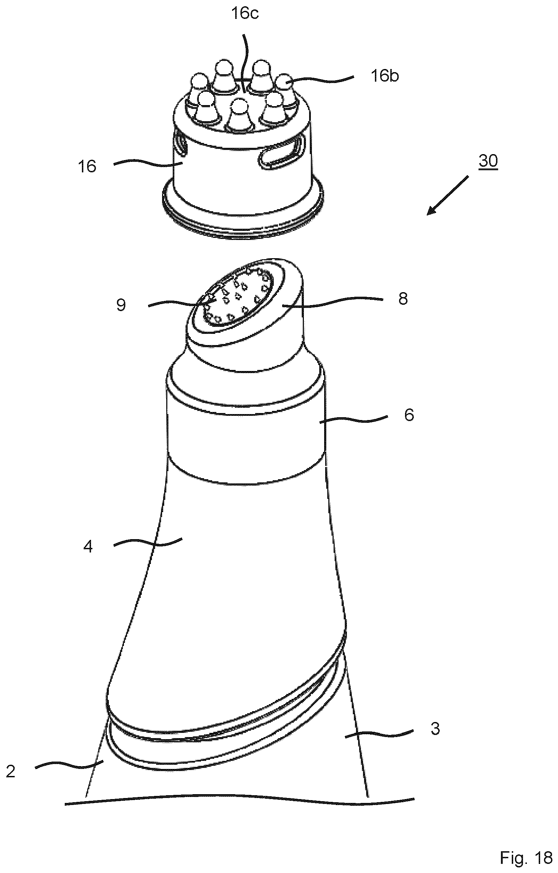

Die Fig. 17 und 18 zeigen das weitere Microneedling-Handgerät 30 mit lösbar aufgesetzter und abgenommener Abdeck- oder Schutzkappe 16. An der Abdeckkappe 16, welche im aufgesetzten Zustand (vgl. Fig. 17) die mehreren nicht-stechenden Stimulationsnadeln 10 abdeckt, ist ein Funktionswerkzeug 16a angeordnet, welches bei dem dargestellten Ausführungsbeispiel mit einer Anordnung von Funktionselementen 16b gebildet ist, bei denen es sich beispielweise um Massageelemente handelt. Die Abdeckkappe 16 ist so als Multifunktionskappe ausgeführt. Beim dargestellten Ausführungsbeispiel sind die Funktionselemente 16b insbesondere im Bereich einer Frontfläche 16c der Abdeckkappe 16 ausgebildet.

Zusätzlich kann die Abdeckkappe 16, zumindest außen, eine geneigte Stirnfläche mit Funktionswerkzeug aufweisen. Auch wegklappbar ist denkbar, oder auch eine axial verschiebliche Hülse mit fehlender oder für das Funktionswerkzeug durchbrochener Deckfläche (vergleichbar einer Art Bohrkrone mit Werkzeugen am Umfang oder auf der Restdeckfläche), zu-/ abschaltbar bspw. durch eine Drehkulisse-Kurvensteuerung per Dreh-Schiebe-Bewegung.

Andere Funktionswerkzeuge können vorgesehen sein, beispielsweise Silikonnoppen, Greif-Kneif-Werkzeuge aus elastischem Material und / oder abwechselnd gerichtete Stimulationswerkzeuge, die ihrerseits eine stech-ähnliche (Stechen simulierend) Wirkung auf der Haut erzielen und hierbei eine Hautspreizung bewirken. Alternative Funktionswerkzeuge zur Hautstimulierung können vorgesehen sein, die sich beispielweise bezüglich eines Spitzenradius oder -winkels von den vorgenannten unterscheiden.

Die Nadelplatte 9 kann in einer Ausgestaltung als Wendeplatte oder Wendeeinsatz ausgeführt sein, derart, dass unterschiedliche Funktionswerkzeuge hieran jeweils wechsel- oder lösbar angeordnet werden, um mehrere Funktionen bereitzustellen oder einen Verschleiß zu kompensieren. Es kann sich auch um Wechseleinsätze handeln, die je nach gewünschter Anwendung an der Nadelplatte 9 lösbar angeordnet werden. Wendeeinsätze können auf beiden Seiten Werkzeuge tragen. Die Wechseleinsätze können aus einem Sortiment oder einem abgestuften Satz von Werkzeugeigenschaften auswählbar sein.

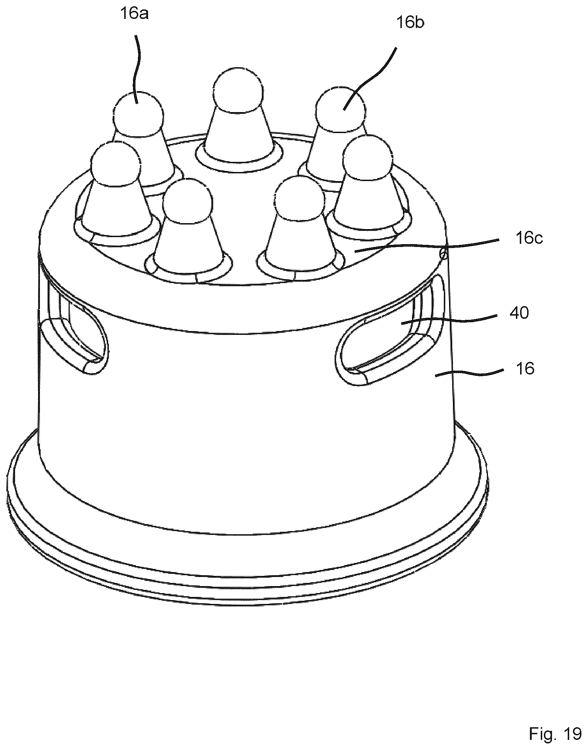

Fig. 19 zeigt eine vergrößerte perspektivische Darstellung der Schutz- oder Abdeckkappe 16 mit dem Funktionswerkzeug 16a. An der Abdeckkappe 16 sind Öffnungen 40 vorgesehen.

Im gezeigten Beispiel sind die Öffnungen im Bereich einer Mantelfläche 41 der Abdeckkappe 16 angeordnet und bilden so seitliche Öffnungen. Alternativ oder ergänzend können solche Öffnungen bei anderen Ausführungsformen im Bereich der Frontfläche 16c angeordnet sein (vgl. Fig. 5). Zum Beispiel können die Öffnungen 40 dazu dienen, für die Nadelplatte 9 mit den mehreren Stimulationsnadeln 10 bei aufgesetzter Abdeckkappe 16 eine Gassterilisation durchzuführen. Die in Fig. 18 und 19 gezeigten seitlichen Öffnungen 40 haben den Vorteil, dass der Eintritt von Staubpartikeln zu der Nadelplatte 9 und hierdurch verursachte Staubablagerungen gemindert sind.

## Patentansprüche

1. Microneedling-Handgerät (1; 20; 30) zum lokalen Stimulieren einer Haut, mit
- einem Gehäuse (2);
- einer Antriebseinrichtung, die in dem Gehäuse (2) angeordnet und eingerichtet ist, eine Antriebskraft bereitzustellen;
- einer Hautstimulationseinrichtung (6);
- einem Microneedling-Stimulationswerkzeug (8), das an der Hautstimulationseinrichtung (6) gebildet und mit der Antriebseinrichtung verbunden ist, derart, dass das Microneedling-Stimulationswerkzeug (8) mittels der Antriebskraft im Betrieb mit einer Wiederholfrequenz vor und zurück bewegbar ist; und
- einer Nadelplatte (9), die an dem Microneedling-Stimulationswerkzeug (8) gebildet ist und bei der über eine vorderseitige Anwendungsfläche (11) verteilt mehrere nicht-stechende Stimulationsnadeln (10) mit einer stumpfen Nadelspitze (10a) angeordnet sind;
wobei das Microneedling-Stimulationswerkzeug (8) mittels der Antriebskraft im Betrieb mit der Wiederholfrequenz zwischen einer vorderen Arbeitsstellung und einer im Vergleich hierzu zurückgestellten hinteren Arbeitsstellung vor und zurück bewegbar ist.

2. Microneedling-Handgerät (1; 20; 30) nach Anspruch 1, **dadurch gekennzeichnet, dass** beim Bewegen zwischen der vorderen Arbeitsstellung und der hinteren Arbeitsstellung zumindest die mehreren nicht-stechende Stimulationsnadeln (10) der Nadelplatte (9) in der vorderen Arbeitsstellung und der hinteren Arbeitsstellung des Microneedling-Stimulationswerkzeugs (8) vollständig außerhalb eines vorderen Gehäuseteils (4) der Hautstimulationseinrichtung des ein- oder mehrstückigen Gehäuses (2) und so freiliegend angeordnet sind.

3. Microneedling-Handgerät (1; 20; 30) nach Anspruch 1 oder 2, dadurch **gekenn**- **zeichnet,** dass im Betrieb weiterhin zumindest die vorderseitige Anwendungsfläche (11) in der vorderen Arbeitsstellung und der zurückgestellten hinteren Arbeitsstellung des Microneedling-Stimulationswerkzeugs (8) vollständig außerhalb des Gehäuses (2) und so freiliegend angeordnet ist,
wobei die Nadelplatte (9) an einem Gehäuseabschnitt (4a) des Gehäuses angeformt ist, welcher im Betrieb zusammen mit dem Microneedling-Stimulationswerkzeug (8) vor und zurück bewegt wird,
wobei insbesondere ein seitlicher Wandabschnitt des Gehäuseabschnitts einstückig mit einer die Anwendungsfläche bereitstellenden Frontplatte ausgeführt ist,
wobei vorzugsweise der Gehäuseabschnitt das Microneedling-Stimulationswerkzeug, insbesondere die Nadelplatte, bildend ausgeführt.

4. Microneedling-Handgerät (1; 20; 30) nach mindestens einem der vorangehenden Ansprüche, **dadurch gekennzeichnet, dass** bei dem Microneedling-Stimulationswerkzeug (8) die Nadelplatte (9) frei von einem die Nadelplatte (9) zumindest teilweise umgreifenden Gehäuseabschnitt des Gehäuses (2) gebildet ist.

5. Microneedling-Handgerät (1; 20; 30) nach mindestens einem der vorangehenden Ansprüche, **dadurch gekennzeichnet, dass** die vorderseitige Anwendungsfläche (11) mit den hierauf verteilt angeordneten mehreren nicht-stechenden Stimulationsnadeln (10), zumindest in der vorderen Arbeitsstellung, zu wenigstens einer Bezugsrichtung aus der folgenden Gruppe schräg gestellt ist: Längsrichtung des Gehäuses (2), Bewegungsrichtung des Microneedling-Stimulationswerkzeugs (8) beim Verlagern zwischen der vorderen und der zurückgestellten hinteren Arbeitsstellung und Bewegungsrichtung der Nadelplatte (9) beim Verlagern zwischen der vorderen und der zurückgestellten hinteren Arbeitsstellung.

6. Microneedling-Handgerät (1; 20; 30) nach mindestens einem der vorangehenden Ansprüche, **gekennzeichnet durch** eine Schutzkappe, die am Gehäuse (2) lösbar und die Nadelplatte (9) abdeckend angeordnet ist, wobei an der Schutzkappe (16) außen ein Funktionswerkzeug angeordnet ist.

7. Microneedling-Handgerät (1; 20; 30) nach mindestens einem der vorangehenden Ansprüche, **dadurch gekennzeichnet, dass** ein vorderes Gehäuseteil (4), welches der Hautstimulationseinrichtung (6) zugeordnet ist, rückwärtig hülsenförmig gestaltet ist und einen hinteren Gehäuseteil (3) zumindest teilweise umschließt, welcher der Antriebseinrichtung zugeordnet ist.

8. Microneedling-Handgerät (1; 20; 30) nach mindestens einem der vorangehenden Ansprüche, **dadurch gekennzeichnet, dass** die Hautstimulationseinrichtung (6) an dem Gehäuse (2) lösbar angeordnet ist.

9. Microneedling-Handgerät (1; 20; 30) nach Anspruch 8, **dadurch gekennzeichnet, dass** die Antriebseinrichtung mit einer Antriebseinrichtung für eine elektrische Zahnbürste gebildet ist, welche eingerichtet ist, hieran wahlweise die Hautstimulationseinrichtung (6) und einen mittels der Antriebseinrichtung betreibbaren Zahnbürstenaufsatz lösbar zu koppeln.

10. Microneedling-Handgerät (1; 20; 30) nach mindestens einem der vorangehenden Ansprüche, **dadurch gekennzeichnet, dass** das Microneedling-Stimulationswerkzeug (8) durch eine Öffnung (21) an einem vorderseitigen Gehäuseende des Gehäuses (2) hindurch in eine im Vergleich zur zurückgestellten hinteren Arbeitsstellung weiter zurückgestellte Nichtarbeitsstellung verlagerbar ist, in welcher zumindest die vorderseitige Anwendungsfläche der Nadelplatte (9) gegenüber der Öffnung (21) zurücksteht.

11. Microneedling-Handgerät (1; 20; 30) nach Anspruch 10, **dadurch gekennzeichnet, dass** die Nadelplatte (9) beim Verlagern durch die Öffnung (21) hindurch ein- oder mehrseitig von einem die Öffnung (21) umgebenden Rand (22) beabstandet ist.

12. Microneedling-Handgerät (1; 20; 30) nach Anspruch 10 oder 11, **dadurch gekennzeichnet, dass** die Nadelplatte (9) in der weiter zurückgestellten Nichtarbeitsstellung beabstandet von die Nadelplatte (9) umgebenden Gehäuseabschnitten des Gehäuses (2) freiliegend angeordnet ist.

13. Microneedling-Handgerät (1; 20; 30) nach mindestens einem der vorangehenden Ansprüche, **gekennzeichnet durch** ein wiederverwendbares Antriebsmodul (4), in welchem die Antriebseinrichtung angeordnet ist, und ein Einwegmodul (7), welches mit dem Antriebsmodul lösbar verbunden ist und in dem die Hautstimulationseinrichtung (6) angeordnet ist.

14. Microneedling-Handgerät (1; 20; 30) nach mindestens einem der vorangehenden Ansprüche, **dadurch gekennzeichnet, dass** die Antriebseinrichtung eingerichtet ist, das Microneedling-Stimulationswerkzeug (8) mittels der hierauf eingeleiteten Antriebskraft im Betrieb mit einer Wiederholfrequenz zwischen etwa 10 Hz und etwa 200 Hz vor und zurück zu bewegen.

15. Hautstimulationseinrichtung (6) mit einem vorderen Gehäuseteil (4). das der Hautstimulationseinrichtung (6) zugeordnet ist, die zum Ausbilden eines Microneedling-Handgeräts (1; 20; 30) nach mindestens einem der vorangehenden Ansprüche an eine Antriebseinrichtung mit einem hinteren Gehäuseteil (3) koppelbar ist, das der Antriebseinrichtung zugeordnet ist, mit:
- einem Microneedling-Stimulationswerkzeug (8), das an der Hautstimulationseinrichtung (6) gebildet und mit der Antriebseinrichtung verbindbar ist, derart, dass das Microneedling-Stimulationswerkzeug (8) mittels einer von der Antriebseinrichtung bereitgestellten Antriebskraft im Betrieb mit einer Wiederholfrequenz vor und zurück bewegbar ist; und
- einer Nadelplatte (9), die an dem Microneedling-Stimulationswerkzeug (8) gebildet ist und bei der über eine vorderseitige Anwendungsfläche (11) verteilt mehrere nicht-stechende Stimulationsnadeln (10) mit einer stumpfen Nadelspitze angeordnet sind;
wobei das Microneedling-Stimulationswerkzeug (8) zwischen einer vorderen Arbeitsstellung und einer im Vergleich hierzu zurückgestellten hinteren Arbeitsstellung vor und zurück bewegbar ist und wobei zumindest die mehreren nicht-stechenden Stimulationsnadeln (10) der Nadelplatte (9) hierbei in der vorderen Arbeitsstellung und der hinteren Arbeitsstellung des Microneedling-Stimulationswerkzeugs (8) vollständig außerhalb des mehrstückigen Gehäuses (2) umfassend das hintere Gehäuseteil (3) und das vordere Gehäuseteils (4) und so freiliegend angeordnet sind.

16. Artikel, der
- eine Antriebseinrichtung, die in einem Gehäuse angeordnet ist, welches wahlweise ein Handstück ausbildend ausgeführt ist;
- einen Zahnbürstenaufsatz, welcher zum Ausbilden einer elektrischen Zahnbürste mit dem Gehäuse lösbar und so an die Antriebseinrichtung koppelnd verbunden werden kann; und
- eine Hautstimulationseinrichtung (6), welche zum Ausbilden eines Microneedling-Handgeräts (1; 20; 30) mit dem Gehäuse lösbar und so an die Antriebseinrichtung koppelnd verbunden werden kann,
aufweist, wobei
- an der Hautstimulationseinrichtung (6) ein Microneedling-Stimulationswerkzeug (8) gebildet ist, das mit der Antriebseinrichtung verbunden ist, derart, dass das Microneedling-Stimulationswerkzeug (8) mittels der Antriebskraft im Betrieb mit einer Wiederholfrequenz vor und zurück bewegbar ist;
- an dem Microneedling-Stimulationswerkzeug (8) eine Nadelplatte (9) gebildet ist, bei der über eine vorderseitige Anwendungsfläche (11) verteilt mehrere nicht-stechende Stimulationsnadeln (10) mit einer stumpfen Nadelspitze (10a) angeordnet sind; und
- das Microneedling-Stimulationswerkzeug (8) mittels der Antriebskraft im Betrieb mit der Wiederholfrequenz zwischen einer vorderen Arbeitsstellung und einer im Vergleich hierzu zurückgestellten hinteren Arbeitsstellung vor und zurück bewegbar ist.

## Claims

1. A handheld microneedling device (1; 20; 30) for locally stimulating a skin, comprising
- a housing (2);
- a drive device arranged in the housing (2) and configured to provide a driving force;
- a skin stimulation device (6);
- a microneedling stimulation tool (8) formed on the skin stimulation device (6) and connected to the drive device such that the microneedling stimulation tool (8) is movable back and forth by means of the driving force at a repetition frequency during operation; and
- a needle plate (9) formed on the microneedling stimulation tool (8) and in which a plurality of non-puncturing stimulation needles (10) with a blunt needle tip (10a) are arranged distributed over a front-side application surface (11);
wherein the microneedling stimulation tool (8) is movable back and forth by means of the driving force at the repetition frequency during operation between a front working position and a rear working position set back in comparison thereto.

2. The microneedling hand-held device (1; 20; 30) according to claim 1, **characterized in that** during movement between the front working position and the rear working position at least the plurality of non-puncturing stimulation needles (10) of the needle plate (9) are arranged, in the front working position and the rear working position of the microneedling stimulation tool (8), completely outside a front housing part (4) of the skin stimulation device of the one-piece or multi-piece housing (2) and thus exposed.

3. The microneedling hand-held device (1; 20; 30) according to claim 1 or 2, **characterized in that** during operation furthermore at least the front-side application surface (11) is arranged, in the front working position and the set-back rear working position of the microneedling stimulation tool (8), completely outside the housing (2) and thus exposed,
wherein the needle plate (9) is formed onto a housing section (4a) of the housing, which is moved back and forth during operation together with the microneedling stimulation tool (8),
wherein in particular a lateral wall section of the housing section is embodied in one piece with a front plate providing the application surface,
wherein preferably the housing section is embodied so as to form the microneedling stimulation tool, in particular the needle plate.

4. The microneedling hand-held device (1; 20; 30) according to at least one of the preceding claims, **characterized in that**, for the microneedling stimulation tool (8), the needle plate (9) is formed free of a housing section of the housing (2) at least partially encompassing the needle plate (9).

5. The microneedling hand-held device (1; 20; 30) according to at least one of the preceding claims, **characterized in that** the front-side application surface (11) with the plurality of non-puncturing stimulation needles (10) arranged distributed thereon is set obliquely, at least in the front working position, with respect to at least one reference direction from the following group: longitudinal direction of the housing (2), movement direction of the microneedling stimulation tool (8) during displacement between the front and the set-back rear working position, and movement direction of the needle plate (9) during displacement between the front and the set-back rear working position.

6. The microneedling hand-held device (1; 20; 30) according to at least one of the preceding claims, **characterized by** a protective cap, which is detachably arranged on the housing (2) and covers the needle plate (9), wherein a functional tool is arranged on the outside of the protective cap (16).

7. The microneedling hand-held device (1; 20; 30) according to at least one of the preceding claims, **characterized in that** a front housing part (4), which is assigned to the skin stimulation device (6), has a sleeve-shaped configuration at the rear and at least partially encloses a rear housing part (3), which is assigned to the drive device.

8. The microneedling hand-held device (1; 20; 30) according to at least one of the preceding claims, **characterized in that** the skin stimulation device (6) is detachably arranged on the housing (2).

9. The microneedling hand-held device (1; 20; 30) according to claim 8, **characterized in that** the drive device is formed with a drive device for an electric toothbrush, which is configured to detachably couple thereto optionally the skin stimulation device (6) and a toothbrush attachment that can be operated by means of the drive device.

10. The microneedling hand-held device (1; 20; 30) according to at least one of the preceding claims, **characterized in that** the microneedling stimulation tool (8) can be displaced through an opening (21) at a front-side housing end of the housing (2) into a non-working position set back further in comparison with the set-back rear working position, in which non-working position at least the front-side application surface of the needle plate (9) is set back with respect to the opening (21).

11. The microneedling hand-held device (1; 20; 30) according to claim 10, **characterized in that** during displacement through the opening (21) the needle plate (9) is spaced apart on one or more sides from an edge (22) surrounding the opening (21).

12. The microneedling hand-held device (1; 20; 30) according to claim 10 or 11, **characterized in that** the needle plate (9) is arranged, in the further set-back non-working position, spaced apart from housing sections of the housing (2) surrounding the needle plate (9) and exposed.

13. The microneedling hand-held device (1; 20; 30) according to at least one of the preceding claims, **characterized by** a reusable drive module (4), in which the drive device is arranged, and a disposable module (7), which is detachably connected to the drive module and in which the skin stimulation device (6) is arranged.

14. The microneedling hand-held device (1; 20; 30) according to at least one of the preceding claims, **characterized in that** the drive device is configured to move the microneedling stimulation tool (8) back and forth by means of the driving force introduced thereon at a repetition frequency between approximately 10 Hz and approximately 200 Hz during operation.

15. A skin stimulation device (6) having a front housing part (4), which is assigned to the skin stimulation device (6), which can be coupled to a drive device having a rear housing part (3), which is assigned to the drive device, in order to form a microneedling hand-held device (1; 20; 30) according to at least one of the preceding claims, comprising:
- a microneedling stimulation tool (8) formed on the skin stimulation device (6) and connectable to the drive device such that the microneedling stimulation tool (8) is movable back and forth by means of a driving force provided by the drive device at a repetition frequency during operation; and
- a needle plate (9) formed on the microneedling stimulation tool (8) and in which a plurality of non-puncturing stimulation needles (10) with a blunt needle tip are arranged distributed over a front-side application surface (11);
wherein the microneedling stimulation tool (8) is movable back and forth between a front working position and a rear working position set back in comparison thereto and wherein at least the plurality of non-puncturing stimulation needles (10) of the needle plate (9) are arranged, in the front working position and the rear working position of the microneedling stimulation tool (8), completely outside the multi-piece housing (2) comprising the rear housing part (3) and the front housing part (4) and thus exposed.

16. An article comprising
- a drive device arranged in a housing, which is optionally embodied so as to form a handpiece;
- a toothbrush attachment, which for forming an electric toothbrush can be detachably connected to the housing and thus coupled to the drive device; and
- a skin stimulation device (6), which for forming a microneedling hand-held device (1; 20; 30) can be detachably connected to the housing and thus coupled to the drive device,
wherein
- a microneedling stimulation tool (8) is formed on the skin stimulation device (6) and connected to the drive device such that the microneedling stimulation tool (8) is movable back and forth by means of the driving force at a repetition frequency during operation;
- a needle plate (9) is formed on the microneedling stimulation tool (8), in which needle plate (9) a plurality of non-puncturing stimulation needles (10) with a blunt needle tip (10a) are arranged distributed over a front-side application surface (11); and
- the microneedling stimulation tool (8) is movable back and forth by means of the driving force at the repetition frequency during operation between a front working position and a rear working position set back in comparison thereto.

## Revendications

1. Appareil manuel de micro-aiguilletage (1 ; 20 ; 30) pour stimuler localement une peau, avec
- un boîtier (2) ;
- un dispositif d'entraînement qui est placé et installé dans le boîtier (2) pour fournir une force motrice ;
- un dispositif de stimulation cutanée (6);
- un outil de stimulation par micro-aiguilletage (8) qui est formé sur le dispositif de stimulation cutanée (6) et relié au dispositif d'entraînement, de telle sorte que d'avant en l'outil de stimulation par micro-aiguilletage (8) puisse être déplacé d'avant en arrière par la force motrice en fonctionnement à une fréquence répétée ; et
- une plaque à aiguilles (9) qui est formée sur l'outil de stimulation par micro-aiguilletage (8) et dans laquelle plusieurs aiguilles de stimulation non piquantes (10) dotées d'une pointe d'aiguille émoussée (10a) sont disposées réparties sur une surface d'application antérieure (11) ;
l'outil de stimulation par micro-aiguilletage (8) pouvant être déplacé d'avant en arrière au moyen de la force motrice en fonctionnement à la fréquence de répétition entre une position de travail avant et une position de travail arrière reculée par rapport à celle-ci.

2. Appareil portatif de micro-aiguilletage (1 ; 20 ; 30) selon la revendication 1, **caractérisé en ce que**, lors du déplacement entre la position de travail avant et la position de travail arrière, au moins les plusieurs aiguilles de stimulation non piquantes (10) de la plaque à aiguilles (9), en position de travail avant et arrière de l'outil de stimulation par micro-aiguilletage (8), sont disposées entièrement à l'extérieur d'une partie avant du boîtier (4) du dispositif de stimulation cutanée du boîtier mono- ou multi-pièces (2) et ainsi de manière à être exposées.

3. Appareil portatif de micro-aiguilletage (1 ; 20 ; 30) selon la revendication 1 ou 2, **caractérisé en ce que**, en fonctionnement, au moins la surface d'application frontale est disposée (11), en position de travail avant et en position de travail arrière reculée de l'outil de stimulation par micro-aiguilletage (8), entièrement à l'extérieur du boîtier (2) et ainsi exposée,
la plaque à aiguilles (9) étant formée sur une section du boîtier (4a) qui est déplacée d'avant en arrière en fonctionnement avec l'outil de stimulation par micro-aiguilletage (8),
une section de paroi latérale de la partie du boîtier étant en particulier réalisée en une seule pièce avec une plaque avant créant la surface d'application ;
la section de boîtier étant de préférence réalisée en formant l'outil de stimulation par micro-aiguilletage, en particulier la plaque à aiguilles.

4. Appareil portatif de micro-aiguilletage (1 ; 20 ; 30) selon au moins une des revendications précédentes, **caractérisé en ce que**, dans l'outil de stimulation par micro-aiguilletage (8), la plaque à aiguilles (9) est formée en étant exempte d'une section de boîtier du boîtier (2) qui embrasse au moins partiellement la plaque à aiguilles (9).

5. Appareil portatif de micro-aiguilletage (1 ; 20 ; 30) selon au moins une des revendications précédentes, **caractérisé en ce que** la surface d'application frontale (11) est inclinée avec les multiples aiguilles de stimulation non piquantes (10) disposées dessus, au moins en position de travail avant, par rapport à au moins un sens de référence du groupe suivant : direction longitudinale du boîtier (2), direction du mouvement de l'outil de stimulation par micro-aiguilletage (8) lors du déplacement entre la position de travail avant et arrière reculée et la direction de mouvement de la plaque à aiguilles (9) lors du déplacement entre la position de travail avant et la position de travail arrière reculée.

6. Appareil portatif de micro-aiguilletage (1 ; 20 ; 30) selon au moins une des revendications précédentes, **caractérisé par** un capuchon de protection qui est disposé de manière amovible sur le boîtier (2) et de manière à couvrir la plaque à aiguilles (9), un outil fonctionnel étant disposé sur l'extérieur du capuchon de protection (16).

7. Appareil portatif de micro-aiguilletage (1 ; 20 ; 30) selon au moins une des revendications précédentes, **caractérisé en ce qu'**une partie avant du boîtier (4) qui est associée au dispositif de stimulation cutanée (6) est conçue à l'arrière en forme de manchon et renferme au moins partiellement une partie arrière du boîtier (3) qui est associée au dispositif d'entraînement.

8. Appareil portatif de micro-aiguilletage (1 ; 20 ; 30) selon au moins une des revendications précédentes, **caractérisé en ce que** le dispositif de stimulation cutanée (6) est disposé de manière amovible sur le boîtier (2).

9. Appareil portatif de micro-aiguilletage (1 ; 20 ; 30) selon la revendication 8, **caractérisé en ce que** le dispositif d'entraînement est constitué d'un dispositif d'entraînement pour une brosse à dents électrique, qui est équipé pour y coupler de manière amovible, au choix, le dispositif de stimulation cutanée (6) et un embout de brosse à dents pouvant être utilisé au moyen du dispositif d'entraînement.

10. Appareil portatif de micro-aiguilletage (1 ; 20 ; 30) selon au moins une des revendications précédentes, **caractérisé en ce que** le dispositif de stimulation par micro-aiguilletage (8) peut être déplacé à travers une ouverture (21) de l'extrémité avant du boîtier (2) vers une position de non-travail plus reculée par rapport à la position arrière de travail reculée dans laquelle au moins la surface d'application frontale de la plaque à aiguilles (9) est en retrait par rapport à l'ouverture (21).

11. Appareil portatif de micro-aiguilletage (1 ; 20 ; 30) selon la revendication 10, **caractérisé en ce que**, lors du déplacement par l'ouverture (21), la plaque à aiguilles (9) est espacée d'un côté ou d'un autre d'un bord (22) entourant l'ouverture (21).

12. Appareil portatif de micro-aiguilletage (1 ; 20 ; 30) selon la revendication 10 ou 11, **caractérisé en ce que** la plaque à aiguilles (9) est disposée de manière exposée, dans la position de non-travail plus reculée, à l'écart des sections de boîtier (2) entourant la plaque à aiguilles (9).

13. Appareil portatif de micro-aiguilletage (1 ; 20 ; 30) selon au moins une des revendications précédentes, **caractérisé par** un module d'entraînement réutilisable (4) dans lequel le dispositif d'entraînement est disposé et un module à usage unique (7), qui est relié au module d'entraînement de manière amovible et dans lequel le dispositif de stimulation cutanée (6) est disposé.

14. Appareil portatif de micro-aiguilletage (1 ; 20 ; 30) selon au moins une des revendications précédentes, **caractérisé en ce que** le dispositif d'entraînement est conçu pour déplacer l'outil de stimulation par micro-aiguilletage (8) d'avant en arrière en fonctionnement avec une fréquence de répétition comprise entre environ 10 Hz et environ 200 Hz, au moyen de la force motrice conduite dessus.

15. Dispositif de stimulation cutanée (6) avec une partie avant du boîtier (4) qui est associée au dispositif de stimulation cutanée (6) qui peut être couplé, pour la formation d'un appareil manuel de micro-aiguilletage (1 ; 20 ; 30) selon au moins une des revendications précédentes, à un dispositif d'entraînement par une partie arrière de boîtier (3) qui est associée au dispositif d'entraînement, avec :
- un outil de stimulation par micro-aiguilletage (8) qui est formé sur le dispositif de stimulation cutanée (6) et pouvant être relié au dispositif d'entraînement, de telle sorte que l'outil de stimulation par micro-aiguilletage (8) puisse être déplacé d'avant en arrière en fonctionnement à une fréquence de répétition au moyen d'une force motrice fournie par le dispositif d'entraînement ; et
- une plaque à aiguilles (9) formée sur l'outil de stimulation par micro-aiguilletage (8) et sur laquelle plusieurs aiguilles de stimulation non piquantes (10) sont disposées réparties sur une surface d'application antérieure (11) avec une pointe d'aiguille émoussée ;
l'outil de stimulation par micro-aiguilletage (8) pouvant être déplacé d'avant en arrière entre une position de travail avant et une position de travail arrière reculée par rapport à celle-ci, et au moins les plusieurs aiguilles de stimulation non piquantes (10) de la plaque à aiguilles (9) étant alors disposées dans la position de travail avant et la position de travail arrière de l'outil de stimulation par micro-aiguilletage (8) complètement hors du boîtier multi-pièce (2) comprenant la partie arrière du boîtier (3) et la partie avant du boîtier (4) et ainsi exposées.

16. Article, qui présente
- un dispositif d'entraînement qui est disposé dans un boîtier qui est au choix conçu pour former une pièce à main ;
- un embout de brosse à dents qui peut être détaché du boîtier pour former une brosse à dents électrique et peut ainsi être relié au dispositif d'entraînement pour établir un couplage ; et
- un dispositif de stimulation cutanée (6), qui peut être détaché du boîtier pour former un appareil manuel de micro-aiguilletage (1 ; 20 ; 30) et qui peut ainsi être relié au dispositif d'entraînement pour établir un couplage,
- étant formé sur le dispositif de stimulation cutanée (6), un outil de stimulation par micro-aiguilletage (8) qui est relié au dispositif d'entraînement, de telle sorte que l'outil de stimulation par micro-aiguilletage (8) puisse être déplacé d'avant en arrière à une fréquence de répétition par la force motrice en fonctionnement ;
- étant formée sur l'outil de stimulation par micro-aiguilletage (8) une plaque à aiguilles (9), sur laquelle plusieurs aiguilles de stimulation non piquantes (10) dotées d'une pointe d'aiguille émoussée (10a) sont disposées réparties sur une surface d'application antérieure (11) ; et
- l'outil de stimulation par micro-aiguilletage (8) pouvant être déplacé d'avant en arrière au moyen de la force motrice en fonctionnement à la fréquence de répétition entre une position de travail avant et une position de travail arrière reculée par rapport à celle-ci.
